# EUROPEAN PATENT APPLICATION

(11) **EP 3 653 214 A1**
(43) Date of publication of application: **20.05.2020**
(21) Application number: 18020612.0
(22) Date of filing: 16.11.2018
(51) Int. Cl.: A61K 31/575, A61P 1/16

(54) **IMPROVED DOSAGE REGIMEN FOR URSODEOXYCHOLIC ACID BASED ON ALKALINE PHOSPHATASE LEVELS IN THE TREATMENT OF CHRONIC CHOLESTATIC LIVER DISEASES**

(71) Applicant: Disphar International B.V., 7255 PZ Hengelo (NL)
(72) Inventor: Van Tomme, Sophie Rolande, 7341 LM Baarn (NL); Gonzales, Rasielle Joy, 7341 LM Baarn (NL); Rodriguez Calvo, Monica, 48150. Sondika (Vizcaya) (ES); Lukas, John, 48160 Derio (Vizcaya) (ES)
(74) Representative: Swarte, Veronica Regina

(57) **Abstract**

The present invention relates to the treatment of chronic cholestatic liver diseases with ursodeoxycholic acid (UDCA) with an improved dosage regimen.

## Description

### Field of the invention

The invention relates to an improved dosage regimen for an pharmaceutical composition comprising ursodeoxycholic acid (UDCA) for use in the treatment of chronic cholestatic liver diseases.

### Background of the invention

Cholestasis is a chronic liver condition resulting from an impairment of the biliary system wherein bile secretion from the liver to the intestine is decreased or interrupted, leading to the degeneration of liver tissues, chronic inflammation and the formation of scar tissue (fibrosis). The consequences are very serious, and if not treated cholestasis will invariably progress towards cirrhosis, hepatic insufficiency, and ultimately liver transplantation.

Primary biliary cholangitis (PBC), previously known as primary biliary cirrhosis is one of the most common chronic cholestatic liver diseases. The incidence of PBC ranges between 0.33 and 5.8 per 100,000 per year, with prevalence rates of 1.91 to 40.2 per 100,000. The ratio of women to men with PBC is 10 to 1. The preclinical phase of the disease is marked by autoimmunity, resulting in slow destruction of small intrahepatic bile ducts. PBC then progresses through three irreversible states: a) cirrhosis; b) a terminal phase defined by high serum bilirubin levels with or without gastrointestinal (GI) bleeding, ascites, or encephalopathy; and c) death unless liver transplant is performed (R. Poupon; J. Hepatol. 2010, 52(5);745-58).

Treatment of PBC with hydrophilic bile acid (BA) ursodeoxycholic acid (UDCA) has been demonstrated to slow the disease progression. Although the mechanism of action is not well understood, UDCA provided proof of concept for BA therapy in PBC. UDCA was proposed as a potential therapy for cholestatic liver disease based on the following rationales: accumulation of toxic bile acids might be at least partially responsible for liver injury in chronic cholestasis, and replacement of endogenous bile acids by a non-toxic bile acid (UDCA) might protect the liver and retard the progression of these disorders. In preliminary studies of patients with PBC, UDCA was found to provide marked improvement in liver tests such as alkaline phosphatase, bilirubin and gamma-glutamyl transferase, leading to randomized trials and meta-analyses that also found a benefit. Hence currently UDCA is the most commonly used drug for primary biliary cirrhosis as it relieves the symptoms and improved the transplant free survival time of patients.

UDCA (ursodeoxycholic acid, ursodiol) was first identified as a major constituent of dried bile of the Chinese black bear and has been used for centuries as remedy for liver diseases in Chinese medicine. UDCA represents up to 3% of the bile acid pool in humans. After oral administration, UDCA is passively absorbed in the small intestine. It is mainly conjugated with glycine and taurine in the human liver and secreted into bile, where it subsequently undergoes enterohepatic circulation caused by active reabsorption of UDCA amides in the terminal ileum.

There are two main originator products in the public domain in Europe, i.e. Ursofalk® from Dr. Falk Pharma and Delursan® from Aptalis Pharma. These two cover a comprehensive series of hepatobiliary disorders, primarily gallstone (dissolution) and primary biliary cirrhosis (PBC) and also primary sclerosing cholangitis (PSC), low phospholipid associated choletithiasis, chronic and genetic cholestasis (LPAC). Ursofalk® is available as 250 mg capsules and 500 mg tablets and Delursan® as tablets at 250 and 500 mg. For patients weighing less than 47 kg or patients who are unable to swallow Ursofalk® capsules, Ursofalk® suspension is also available. The drug is administered in adults and children.

The following daily dose amount is indicated in the summary of product characteristics (SmPC) to be used as standard for the treatment of Primary biliary cirrhosis (PBC). The daily dose depends on body weight, and ranges from 750 mg to 1750 mg UDCA, i.e. 14 ±2 mg ursodeoxycholic acid per kg of body weight. For the first 3 months of treatment, UDCA should be taken divided over the day. With improvement of the liver values the daily dose may be taken once daily in the evening, but the daily dose amount itself stats the same.

Once PBC is diagnosed, the use of UDCA may be continued indefinitely. Daily dose amount may exceed 2000 mg in 3 x daily administration of possibly more than one tablet each time. No prevision is described for these products accounting for the potential of overdose and adverse events also due to an individually elevated baseline and the related reduction in absorption of the drug.

Non-response or sub-optimal response to UDCA is common in certain types of patients. It is known that patients with high serum bilirubin levels (more than 3 mg/L), high serum alkaline phosphatase (ALP) activity, and cholesterol have usually severe ductopenia at presentation (the premature ductopenic variant of PBC) and will not respond to any medical therapy, including treatment with UDCA. Overall it is accepted by the opinion leaders of PBC research in Europe that approximately 40% of patients do not achieve at least one condition of optimal biochemical response with the current approved dosage regimen, and can therefore be classified as sub-optimal responders to UDCA (R. Poupon; J. Hepatol. 2010, 52(5);745-58). Although causes of suboptimal response are not yet clear, reduced absorption and poor compliance are speculated.

It is reported that after treatment with UDCA at doses corresponding to 4 mg/kg/day, the liver function indices such as alkaline phosphatase (ALP), gamma glutamyl transferase (GGT), alanine aminotransferase (ALT) and aspartate aminotransferase (AST) were significantly lower than pretreatment values. A further significant reduction during treatment with 500 mg/day and 750 mg/day was observed for ALP and gamma glutamyl transpeptidase (γ-GTP) (Podda et all; Dig. Dis Sci. 1989, Dec. 34 (12 Suppl.): 59S-65S). Improvement is significant after 4 to 8 weeks in a weakly dose-dependent manner.

Moreover, serum ALP is currently used as an indirect marker of cholestasis. Evidence also indicates that serum ALP levels are the primary marker of long-term prognosis of PBC and the therapeutic response to UDCA treatment in both PBC and PSC (R. Poupon; Hepatology; 2015 61(6):2080-2090).

Although UDCA is generally well tolerated, the major issue with the life-time use of UDCA - especially when used in high amounts - are the side-effects such as headache, weight gain, constipation and diarrhea. Given the fact that PBC patients already suffer from gastrointestinal disorders, such side-effects are highly undesirable.

To this end, the present invention is directed to a more adequate UDCA dosage regimen.

### Summary of the invention

It is an object of the present invention to provide a new dosage regimen for an UDCA formulation for use in the treatment of chronic cholestatic liver diseases.

Said dosage regimen is based on an individualization of the UDCA dosage amount by measuring an alkaline phosphatase level in the patient rather than the standard use of body weight-based dosing.

Hence, in one embodiment, the present invention provides for a pharmaceutical composition comprising ursodeoxycholic acid (UDCA) for use in the treatment of a chronic cholestatic liver disease in a patient, characterized in that a daily dose amount of UDCA is administered to the patient that is based on an alkaline phosphatase (ALP) level of said patient. In the present invention, patient's ALP level, as can be measured in the blood, determines the amount of UDCA that is given daily to said patient to treat a chronic cholestatic liver disease.

In one embodiment, the present invention provides a method for treatment of a chronic cholestatic liver disease in a patient comprising
i.measuring ALP level of the patient (in IU/L) and
ii.determining a daily dose amount of UDCA based on said measured ALP level and
iii. dosing said daily dose amount of UDCA to said patient.

In yet another embodiment the present invention provides a pharmaceutical composition for use, wherein the daily dose amount of UDCA is determined by the ALP level according to the function: UDCA (mg)= 593,6 x e^{(0.00076 x ALP)}, wherein ALP is expressed in IU/L, and optionally, said determined daily dose amount of UDCA is adjusted to a practical daily dose amount that is commercially available. In this function (or mathematical formula), the term e is a mathematical constant that is approximately equal to 2.71828. In the content of the present invention, with a practical daily dose amount is meant an amount of a dosage form that is commercially available and which is closest to the determined daily dose amount. This can be a whole dose or a partial or divided dose in case the dosage form can easily be divided. In an alternative embodiment, a pharmaceutical composition for use is provided wherein said practical daily dose amount of UDCA is plus or minus 20%, preferably 10%, more preferably 5% of the determined UDCA (mg). In yet another embodiment, a pharmaceutical composition for use is provided wherein said practical daily dose amount of UDCA is plus or minus 10 to 250 mg UDCA.

In yet another embodiment, the present invention provides a pharmaceutical composition for use, wherein:
i. when ALP level is between 200-699 IU/L; the daily dose amount of UDCA is between 700 to 1050 mg, preferably it is 700 mg or more, more preferably it is 750 mg or more, or preferably it is 1050 mg or less, more preferably it is 1000 mg or less; or
ii. When the ALP level is between 700-999 IU/L the daily dose amount of UDCA is between 1050-1400 mg; preferably it is 1100 mg or more, more preferably it is 1250 mg or more, or preferably it is 1400 mg or less or more preferably it is 1250 mg or less; or
iii. When the ALP level is between 1000-1400 IU/L, the daily dose amount of UDCA is between 1400 and 1750 mg; preferably it is 1400 mg or more, more preferably it is 1500 mg or more or preferably it is 2000 mg or less or more preferably it is 1750 mg or less.

In cases where the ALP level is above 1400 IU/L, the daily dose amount of UDCA is 1750 mg or increased to 2000 mg in case no reduction in ALP level is observed over a longer period of time.

In one aspect of the invention, it is provided for a pharmaceutical composition comprising UDCA for use in the treatment of a chronic cholestatic liver disease in a patient, characterized in that a daily dose amount of UDCA is reduced when said patient's ALP level is declined.

In yet another aspect, the reduction is:
i. When ALP level is between 200-699 IU/L, the daily dose amount of UDCA is between 700 -1050 mg, preferably it is 700 mg or more, more preferably it is 750 mg or more, or preferably it is 1050 mg or less, more preferably it is 1000 mg or less;
ii. When ALP level is between 700-999 IU/L, the daily dose amount of UDCA is between 1050-1400 mg, preferably it is 1100 mg or more, more preferably it is 1250 mg or more, or preferably it is 1400 mg or less or more preferably it is 1250 mg or less;; and wherein this amount is reduced to the daily dose amount mentioned under (i) once the ALP level is below 700 IU/L;
iii. When ALP level is between 1000-1400 IU/L, the daily dose amount of UDCA is between 1400 and 1750 mg, preferably it is 1400 mg or more, more preferably it is 1500 mg or more or preferably it is 2000 mg or less or more preferably it is 1750 mg or less; and wherein this amount is reduced to the daily dose amount mentioned under (ii) once the ALP level is below 1000 IU/L.

In one embodiment, the present invention provides a method for treatment of a chronic cholestatic liver disease in a patient comprising
a. dosing said patient with a daily dose amount of UDCA based upon an ALP level (in IU/L); and
b. reducing said daily dose amount of UDCA when said patient's ALP level is declined.

In yet another embodiment, a pharmaceutical composition for use is provided wherein the daily dose amount of UDCA is administered once-daily over the whole period of the treatment. Preferably, the UDCA is administered in the evening.

It is yet another embodiment of the present invention to provide a pharmaceutical composition for use comprising 700 mg UDCA, for use in the treatment according to claim 1.

In yet another embodiment, the chronic cholestatic liver disease is primary biliary cirrhosis (PBC) and also primary sclerosing cholangitis (PSC).

It is yet another embodiment of the present invention, to provide for a kit of parts comprising the pharmaceutical composition for use according to the invention, and a patient information leaflet with instructions for enabling the use in a method for treating chronic cholestatic liver diseases in a patient, characterized in that the composition is dosed based on an alkaline phosphatase (ALP) level of said patient

### Description of the Figures

**Figure 1:** Figure 1 shows the relation between the level of ALP (IU/L) as measured in a patient and the amount of UDCA (mg) that should be dosed daily to said patient.
**Figure 2:** Figure 2 shows the decrease in ALP level (IU/L) in patients that are treated in the Podda 1989 trial as well as in the model simulation over the period of 8 weeks treatment with similar daily dose amounts of UDCA (mg).

### Detailed description of the invention

The present invention provides a new dosage regimen for pharmaceutical UDCA compositions that provides less side effects in a patients and which also may reduce the percentage of non-responders to UDCA for the treatment of chronic cholestatic liver diseases such as primary sclerosing cholangitis (PSC) and primary sclerosing cholangitis (PBC).

In the new dosage regimen of the present invention, the daily dose amount of UDCA that is to be given to a patient is determined based on the ALP level (IU/L) in the blood of said patient, instead of being based on the body weight of said patient as is current standard practice. Hence it is an object of the present invention to provide a method for treatment of a chronic cholestatic liver disease in a patient comprising
i. measuring ALP level of the patient (in IU/L) and
ii.determining a daily dose amount of UDCA based on said measured ALP level and
iii. dosing said daily dose amount of UDCA to said patient.

In one embodiment of the present invention, a pharmaceutical composition comprising ursodeoxycholic acid is provided for use in the treatment of a chronic cholestatic liver disease in a patient, characterized in that a daily dose amount of UDCA is administered to the patient that is based on an alkaline phosphatase (ALP) level of said patient.

The inventors surprisingly have found that when UDCA is dosed based on the ALP level, the daily dose amount of UDCA can be highly reduced when compared to dosing based on body weight. Especially in the case of patients that have high body weight but low ALP level, the amount of UDCA based on bodyweight is overdosed. Overdosing may result in unnecessary adverse events. On the other hand, a slim patient with high ALP levels is underdosed with the current approach using body weight. Underdosing may result in sub-optimal therapy or non-response.

Additionally, with the proposed dosage regimen, the total amount of UDCA intake for a patient over a year is highly reduced since said UDCA intake will be tapered upon decreasing ALP levels, whereas the currently established dosage regimen will be maintained unaltered and unnecessarily elevated.

From a historical point of view regarding the persistence of weight-based dosing, UDCA was first approved for the treatment of radiolucent gallstones with doses given in a mg/kg basis. Early studies with UDCA for PBC treatment tested the already approved UDCA dosing for gallbladder and several studies were conducted later, most of them following the same mg/kg dose approach. Based on those studies UDCA was later approved for PBC, maintaining the dosing given in a mg/kg basis. However, there is no solid evidence that weight has an impact on the treatment of PBC.

Alkaline phosphatase (ALP) is an enzyme that can be found and measured in blood serum and it exists in different forms, depending on where it originates. Isoenzyme ALP-1 is found primarily in the liver and isoenzyme ALP-2 in the bone. There are also small amounts produced by cells lining the intestines (isoenzyme ALP-3), the placenta, and the kidney (in the proximal convoluted tubules). ALP is a hydrolase enzyme responsible for dephosphorylation of many types of molecules, including nucleotides, proteins, and alkaloids. The primary importance of measuring ALP levels is to check the possibility of bone disease or liver disease. ALP levels in serum rise with large bile duct obstruction, intrahepatic cholestasis, or infiltrative diseases of the liver. ALP is not specific for biliary tract disease but is standardly tested in patients that are diagnosed with PBC. The test to measure the level of ALP in the bloodstream requires a simple blood draw from a patient and is a routine part of other liver panel blood tests. The ALP level test is a standardly performed laboratory test using a commercially available assay kit for quantitative determination of alkaline phosphatase activity in serum or plasma. Said kits comprise internal reference standards that guarantee the accuracy of the assay. The reference range of the ALP level in healthy adult subjects is from 20 to 140 IU/L. In the content of the present invention, ALP level means the amount of ALP activity in international units per liter (IU/L or U/L) in blood as measured using a standard laboratory test kit. When ALP values are > 1.5 times the upper limit of normal (ULN) this is an identification of a liver disease. ALP is used to monitor PBC disease progression and also to conclude if a patient is a UDCA non responder.

Although ALP level is currently seen as an indirect marker for the long-term prognosis of PBC (R. Poupon; Hepatology; 2015 61(6):2080-2090), this document does not teach nor suggest that the daily dose amount of UDCA (in mg) that should be given to treat a patient, is to be calculated based on the level of ALP (in IU/L) in said patient.

Hence, for the current invention, a large amount of publicly available pharmacodynamic (PD) data and clinical outcomes were analysed. Exploratory data analysis (EDA) involving extensive graphical exploration and validation of the received data, and diagnostic preliminary statistics was performed relevant to model development and building of a standardized population model. Additional, properties relating to UDCA pharmacokinetics (PK) were observed in EDA to guide modeling. Several biomarkers such as biliary enrichment (BE), Aspartate Transaminase (AST), Alanine Transaminase (ALT), Gamma Glutamyl transferase (GGT), and alkaline phosphatase (ALP) were explored with the objective to arrive at quantifying the relation with UDCA dosing in a computer simulation meta-data model.

Surprisingly the meta-analysis showed that at start of treatment in patients with chronic cholestatic liver diseases, the ALP level is the most discriminating predictor of response to a given UDCA dose. Additionally, it was shown that a higher ALP level would require higher UDCA doses, regardless of the patient weight.

Hence it is an object of the present invention to establish an appropriate daily dose amount of UDCA based upon patient's ALP level (IU/L).

In the content of the present invention, based on an ALP level means that the ALP level (IU/L) as measured in a patient is the determining factor that forms the basis for the amount of UDCA that is to be given to a patient.

Hence, in yet another embodiment of the present invention, it is provided for a pharmaceutical composition for use wherein the daily dose amount of UDCA (mg) is determined by a function of the ALP level according to the formula: UDCA (mg)= 593,6 x e^{(0.00076 x ALP)}, wherein ALP is expressed in IU/L. Optionally, said daily dose amount is adjusted to a practical amount commercially available. In this embodiment, the daily dose amount of UDCA is a function of the ALP level that results in an amount of UDCA that is very precise (several figures behind the comma) and not practical to be dosed with current available commercial products. Hence, for convenient and practical reasons, the determined amount of UDCA according to the formula may optionally be adjusted to an amount that is practically in view of the commercial products available in the market. This amount may be the nearest whole dose in case of UDCA capsules, or a partly dose in case of UDCA tablets (f.e. a half tablet) or a liquid dose (metered dose). In case the ALP level is 200 IU/L, then the determined UDCA daily dose amount according to the formula is about 691 mg. In case a 700 mg tablet is commercially available, optionally a practical dose of 700 mg may be dosed. As an example, in case the measured ALP level in a patient is 1300 IU/L, then the determined UDCA daily dose amount according to the formula is about 1594 mg. In case a 500 mg tablet is commercially available, optionally a practical dose of 3 times the 500 mg tablet corresponding to 1500 mg may be dosed.

In comparison to prior art dosing based on bodyweight (e.g. 13 mg/kg), a 50 kg patient would have received 650 mg, while a patient with 85 kg would have received 1105 mg. Hence, if both patients have the same ALP level such as for example 700 IU/L (resulting in a daily dose of 1011 mg UDCA according to the invention), the 50 kg patient would be under-dosed with 650 mg UDCA whereas on the other hand, the 85 kg patient would have been a little overdosed with 1105 mg.

In yet another embodiment, and in view of the UDCA products as currently commercially available, a more practical dosing scheme is applied:
A pharmaceutical composition for use according to claim 1, wherein:
iv. When ALP level is between 200-699 IU/L; the daily dose amount of UDCA is between 700 to 1050 mg, or
v. When the ALP level is between 700-999 IU/L the daily dose amount of UDCA is between 1050-1400 mg; or
vi. When the ALP level is between 1000-1400 IU/L, the daily dose amount of UDCA is between 1400 and 1750 mg.

The dose given depends on alkaline phosphatase (ALP) levels at entry (baseline) and is adjusted as treatment proceeds.

The time needed for an important reduction in ALP is approximately 8 weeks and depends on the previous history of the patient and their disease severity.

The monitoring of ALP level in a patient may vary from several weeks to several months, depending on the severity of the disease or depending on hospital guidelines or on the standard protocol that the physician uses. However, for higher ALP levels as well as other hepatobiliary enzymes, initial assessment is recommended every 4 weeks. When the ALP level changes, the appropriate daily dose amount of UDCA that is to be dosed, changes accordingly. A dose adjustment (reduction) can continue until two consecutive assessments at an interval of at least 8-12 weeks shows stabilization of the ALP level. In case of stabilization, the treatment can continue chronically at a fixed dose level with yearly ALP assessments.

Preferably, the ALP level is to be measured just before establishing a first, initial daily dose amount of UDCA that is to be given at the start of the disease (base line ALP level). Additionally the ALP level is monitored regularly for establishing an appropriate subsequent dose of UDCA during the progression of the disease. Also patients that have previously received a daily dose amount of UDCA that was based upon body weight, may start using a UDCA daily doseamount that is based on the ALP level.

In one embodiment, the present invention provides a method for treatment of a chronic cholestatic liver disease in a patient comprising
a. dosing said patient with a daily dose amount of UDCA based upon an ALP level (in IU/L); and
b. reducing said daily dose amount of UDCA when said patient's ALP level is declined.

In one embodiment it is provided for, a pharmaceutical composition comprising UDCA for use in the treatment of a chronic cholestatic liver disease in a patient, characterized in that a daily dose amount of UDCA is reduced when said patient's ALP level is declined.

In yet another embodiment, the reduction is:
a. When ALP level is between 200-699 IU/L, the daily dose amount of UDCA is between 700 -1050 mg;
b. When ALP level is between 700-999 IU/L, the daily dose amount of UDCA is between 1050-1400 mg; and wherein this amount is reduced to the daily dose amount mentioned under (i) once the ALP level is below 700 IU/L;
c. When ALP level is between 1000-1400 IU/L, the daily dose amount of UDCA is between 1400 and 1750 mg; and wherein this amount is reduced to the daily dose amount mentioned under (ii) once the ALP level is below 1000 IU/L.

It is currently standard procedure in UDCA treated patients, that during the first 3 months of treatment, liver function parameters alkaline phosphatase (ALP), AST (SGOT), ALT (SGPT) and γGT are monitored by the physician every 4 weeks, thereafter every 8 to 12 weeks. Apart from allowing for identification of responders and non-responders in patients being treated for PBC, this monitoring also enables early detection of potential hepatic deterioration, particularly in patients with advanced stage PBC.

Without being bound by theory, it is believed that with the new dosing regimen of the current invention, the amount of non-responders may drastically decrease and so would drug-related adverse events in overdosed patients.

In the present invention, with the term daily dose amount of UDCA is meant the amount of UDCA that is given to a patient on a single day (24 hours). The amount of UDCA can be expressed in milligrams (mg). The daily dose amount can be given every day or it can be given with time-intervals. Standard practice with currently available commercially products is dosing every day during the progression of the disease.

In yet another aspect of the present invention, the daily dose amount can be divided to be taken in the morning and/or, midday and/or evening. Moreover, in the currently marketed products, the daily UDCA intake is divided over multiple doses since this was established to be the most appropriate approach to maximise the biliary enrichment and therefore cost effective. Only after improvement of the liver values, the daily dose amount may be taken once daily in the evening. Surprisingly, it was shown in the present invention that a single daily dose amount is as beneficial as a divided intake already at start of treatment. This once-daily intake is an important advantage since non-compliance is also speculated to be a cause of sub-optimal response. The daily dose amount may preferably be taken in the evening. Hence, it depends on the patient preference, in combination with the dose amount that is the most suitable.

The pharmaceutical UDCA composition for use according to the invention, can be any currently available tablet or capsule and granulate or suspension or the like, and in any available strength. Preferably, a 700 mg composition is used. This composition can be manufactured according to state of the art technology.

In the present invention, the chronic cholestatic liver disease is preferably PBC or PSC. However, without being bound to theory, any already existing or future disease that requires UDCA treatment, may be comprised within the scope of this invention. This may include cystic fibrosis-related liver disease.

The pharmaceutical product for use according to the present invention may be commercialized as a kit of parts comprising the UDCA pharmaceutical composition packaged together with a patient information leaflet (PIL) with instructions for enabling the use in a method for treating chronic cholestatic liver diseases in a patient, characterized in that the composition is dosed based on the alkaline phosphatase (ALP) level of said patient.

However, also commercially available products comprising UDCA compositions that do not explicitly contain a PIL with instructions for said ALP-based dosing are considered to be comprised within the scope of the invention, in case the physician uses said UDCA composition for ALP-based dosing.

### EXAMPLES

### Example 1: Modelling

The PK data available from state of the art experimental observations and meta-data for UDCA clinical evidence pharmacodynamics (PD) were extracted and analysed. Established methods were applied to perform modeling and simulation according to standards for modeling in the industry.

All manipulation, statistical regression, graphical exploration and data stream building are done in traceable S+ scripting using S-Plus (Tibco Software, Palo Alto, CA, USA). A semi-physiological PK model for UDCA was built in the nonlinear mixed effects industry-standard method NONMEM® (Icon Plc. Dublin, Ireland). The PK model was linked to the PD (from meta-data) to drive clinical response (ALP reduction) via a physiological prior link to biliary enrichment. The following mathematical representation can be extracted between the level of ALP (IU/L) that is measured in a patient and the amount of UDCA (mg) that is to be daily dosed to said patient: UDCA (mg) = 593,6 x e^{(0.00076 xALP)} (wherein ALP is the ALP level in IU/L).

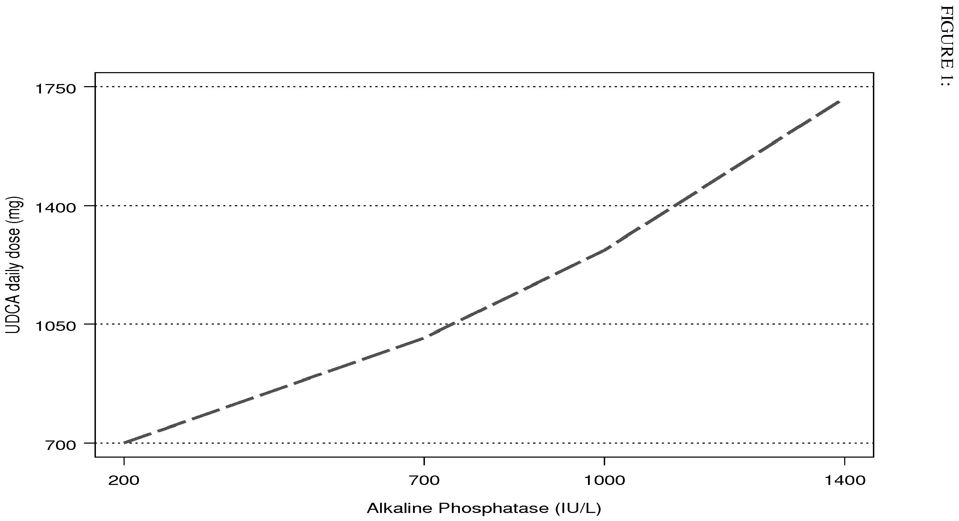

This mathematical representation is illustrated in Figure 1.

### Example 2: Model verification

The PK/PD model as developed above was checked versus public domain data from the Podda 1989 trial (note that the model has been developed independent of this data) in order to show that simulation/prediction with the obtained model matches the given prior art clinical data.

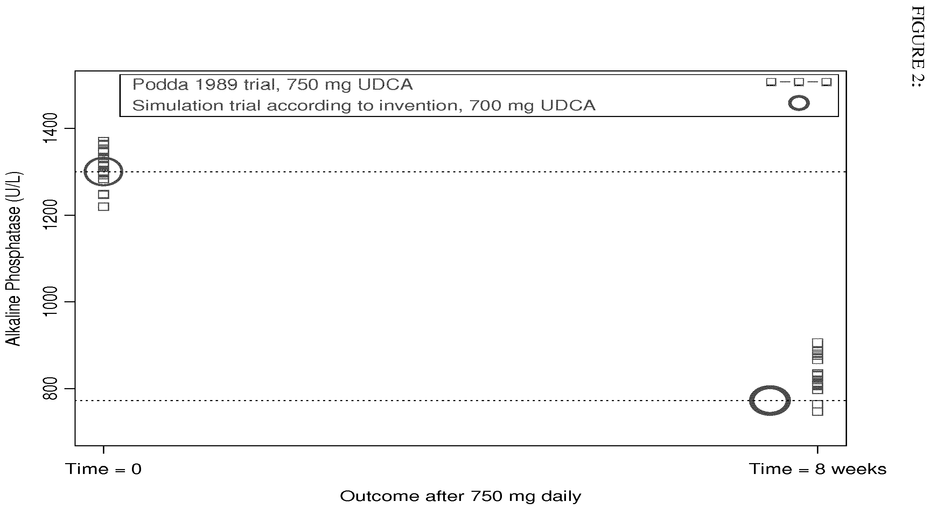

In said Podda 1989 trial, 20 PBC patients -having an ALP level of about 1300 IU/L at the start of the trial- were treated with 750 mg UDCA daily, said doses being based upon their weight, for a period of 8 weeks. After treatment, the ALP values were again measured. The model simulation was based on similar parameters. In Figure 2, the decrease in ALP level (IU/L) is shown for both the Podda 1989 trial as well as the model simulation (simulation trial) over the period of 8 weeks treatment. From the results obtained, it can be concluded that the model as developed and used for determining the dosage regimen, i.e. the daily dose amount of UDCA based on ALP level of the present invention matches the individual prior art data as shown in the Podda 1989 trial.

## Claims

1. A pharmaceutical composition comprising ursodeoxycholic acid (UDCA) for use in the treatment of a chronic cholestatic liver disease in a patient, **characterized in that** a daily dose amount of UDCA is administered to the patient that is based on an alkaline phosphatase (ALP) level of said patient.

2. The pharmaceutical composition for use according to claim 1, wherein the daily dose amount of UDCA (mg) is determined by a function of the ALP level according to the formula:
UDCA (mg)= 593,6 x e^{(0.00076 x ALP)}, wherein ALP is expressed in IU/L,
and optionally, said daily dose amount is adjusted to a practical daily dose amount which is commercially available.

3. The pharmaceutical composition for use according to claims 1 or 2, wherein
i. When ALP level is between 200-699 IU/L, the daily dose amount of UDCA is between 700 to 1050 mg, or
ii. When the ALP level is between 700-999 IU/L, the daily dose amount of UDCA is between 1050-1400 mg; or
iii. When the ALP level is between 1000-1400 IU/L, the daily dose amount of UDCA is between 1400 and 1750 mg.

4. A pharmaceutical composition comprising UDCA for use in the treatment of a chronic cholestatic liver disease in a patient having an ALP level (IU/L), **characterized in that** a daily dose amount of UDCA is reduced when said patient's ALP level is declined.

5. The pharmaceutical composition for use according to claim 4, **characterized in that** the UDCA reduction is :
i. When ALP level is between 200-699 IU/L, the daily dose amount of UDCA is between 700 -1050 mg;
ii. When ALP level is between 700-999 IU/L, the daily dose amount of UDCA is between 1050-1400 mg; and wherein this amount is reduced to the daily dose amount mentioned under (i) once the ALP level is below 700 IU/L;
iii. When ALP level is between 1000-1400 IU/L, the daily dose amount of UDCA is between 1400 and 1750 mg; and wherein this amount is reduced to the daily dose amount mentioned under (ii) once the ALP level is below 1000 IU/L.

6. The pharmaceutical composition for use according to any of claims 1 to 5, wherein the daily dose amount of UDCA is administered once-daily over the whole period of the treatment.

7. The pharmaceutical composition for use according to any of claims 1 to 6 , wherein the UDCA is administered in the evening.

8. A pharmaceutical composition for use comprising 700 mg UDCA, for use in the treatment according to claim 1.

9. The pharmaceutical composition for use according to any of claims 1 to 8, wherein the chronic cholestatic liver disease is primary biliary cirrhosis (PBC) and also primary sclerosing cholangitis (PSC)

10. A kit of parts comprising the pharmaceutical composition for use according to any of claims 1 to 9, and a patient information leaflet with instructions for enabling the use in a method for treating chronic cholestatic liver diseases in a patient, **characterized in that** the composition is dosed based on an alkaline phosphatase (ALP) level of said patient.

11. A method for treatment of a chronic cholestatic liver disease in a patient comprising
i. measuring ALP level of the patient (in IU/L) and
ii. determining a daily dose amount of UDCA based on said measured ALP level and
iii. dosing said daily dose amount of UDCA to said patient.

12. A method for treatment of a chronic cholestatic liver disease in a patient comprising
i. dosing said patient with a daily dose amount of UDCA based upon an ALP level (IU/L);
ii. and reducing said daily dose amount of UDCA when said patient's ALP level is declined.
